(19) Europäisches Patentamt
European Patent Office
Office européen des brevets

(11) Publication number: 0 095 292
A1

# (12) EUROPEAN PATENT APPLICATION

(21) Application number: 83302735.2

(22) Date of filing: 16.05.83

(51) Int. Cl.³: C 07 H 19/06
C 07 H 19/08, A 61 K 31/70

(30) Priority: 22.05.82 GB 8215018
18.08.82 GB 8223846
12.02.83 GB 8303925

(43) Date of publication of application:
30.11.83 Bulletin 83/48

(84) Designated Contracting States:
CH DE FR GB IT LI NL

(71) Applicant: BEECHAM GROUP PLC
Beecham House Great West Road
Brentford Middlesex(GB)

(72) Inventor: Luk, Kong
4 Palmer Close
Horley Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) 5-(2-halogenovinyl)-2'-deoxyuridine derivatives, processes for their preparation, pharmaceutical compositions containing them, and their use in treating viral infections".

(57) Compounds of formula (I):

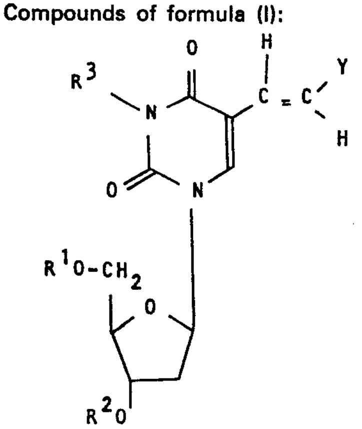

(I)

wherein each of $R^1$, $R^2$ and $R^3$ is a hydrogen atom, an acyl group, or an optionally acylated 5-(*E*-2-halogenovinyl)-2'-deoxyuridinylcarbonyl group, or $R^1$ is a 5-(*E*-2-halogenovinyl)-2'-deoxyuridinylcarbonyl group linked to the 5'-oxygen atom via an alkylene-carbonyl group having from 1 to 8 carbon atoms in the alkylene moiety, and Y is a halogen atom, preferably a bromine atom, and is identical to the halogen atom in the deoxyuridinylcarbonyl or linked deoxyuridinylcarbonyl group, with the provisos that, (a) only one of $R^1$, $R^2$ and $R^3$ is an optionally acylated 5-(*E*-2-halogenovinyl)-2'-deoxyuridinylcarbonyl group, or (b) $R^1$ is a 5-(*E*-2-halogenovinyl)-2'-deoxyuridin-5'-ylcarbonyl group linked via the alkylene-carbonyl group and $R^2$ and $R^3$ are both simultaneously hydrogen atoms, are useful in the treatment of viral infection.

EP 0 095 292 A1

# 5-(2-HALOGENOVINYL)-2'-DEOXYURIDINE DERIVATIVES, PROCESSES FOR THEIR PREPARATION, PHARMACEUTICAL COMPOSITIONS CONTAINING THEM, AND THEIR USE IN TREATING VIRAL INFECTIONS

This invention relates to certain deoxyuridine compounds which have antiviral activity.

European Published Patent Application No. 61,283 discloses certain mono- and di-esters of 5-(E-2-bromovinyl)-2'-deoxyuridine which have antiviral activity, particularly against herpes viruses.

We have now found a group of novel mono-, di- and tri-substituted 5-(2-halogenovinyl)-2'-deoxyuridines which have antiviral activity, and which are useful in the treatment of infections caused by herpes viruses, such as herpes simplex type 1, herpes simplex type 2 and varicella.

According to the present invention there is provided a compound of formula (I):

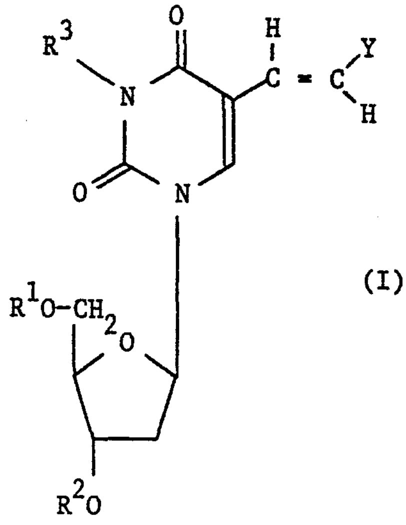

(I)

wherein each of $R^1$, $R^2$ and $R^3$ is a hydrogen atom, an acyl group, or an optionally acylated 5-(E-2-halogeno-vinyl)-2'-deoxyuridinylcarbonyl group, or $R^1$ is a 5-(E-2-halogenovinyl)-2'-deoxyuridinylcarbonyl group linked to the 5'-oxygen atom via an alkylene-carbonyl group having from 1 to 8 carbon atoms in the alkylene moiety, and Y is a halogen atom, preferably a bromine atom, and is identical to the halogen atom in the deoxyuridinylcarbonyl or linked deoxyuridinylcarbonyl group, with the provisos that, (a) only one of $R^1$, $R^2$ and $R^3$ is an optionally acylated 5-(E-2-halogenovinyl)-2'-deoxyuridinylcarbonyl group, or (b) $R^1$ is a 5-(E-2-halogenovinyl)-2'-deoxyuridin-5'-ylcarbonyl group linked via the alkylene-carbonyl group and $R^2$ and $R^3$ are both simultaneously hydrogen atoms.

Suitable acyl groups are those of the formula (II):

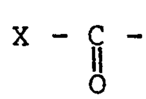

(II)

in which X is an alkyl, aryl or aralkyl group.

Suitably X is a $C_{1-6}$ alkyl, optionally substituted phenyl or optionally substituted benzyl group.

The alkylene moiety in the alkylene-carbonyl group may be a straight or branched chain, but is preferably a straight chain.

Preferred compounds of formula (I) are those wherein $R^2$ and $R^3$ are both simultaneously hydrogen.

Compounds of formula (I) wherein $R^3$ and $R^2$ are hydrogen atoms and $R^1$ is a deoxyuridinylcarbonyl group, may be prepared by reacting a compound of formula (III):

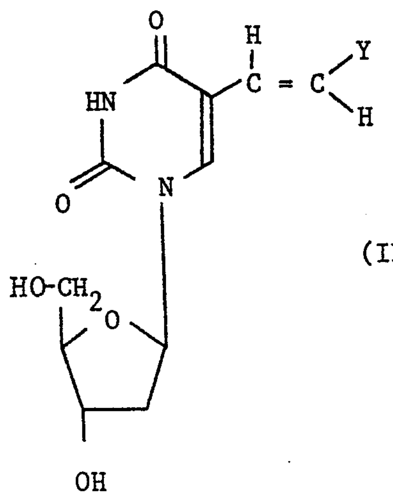

(III)

wherein Y is as defined in formula (I), with phosgene, suitably in an anhydrous polar organic solvent, such as pyridine. The product may be purified chromatographically on silica gel.

Compounds of formula (I) wherein $R^3$ and $R^1$ are hydrogen atoms, and $R^2$ is a deoxyuridinylcarbonyl group, may be prepared by treating a compound of formula (IV):

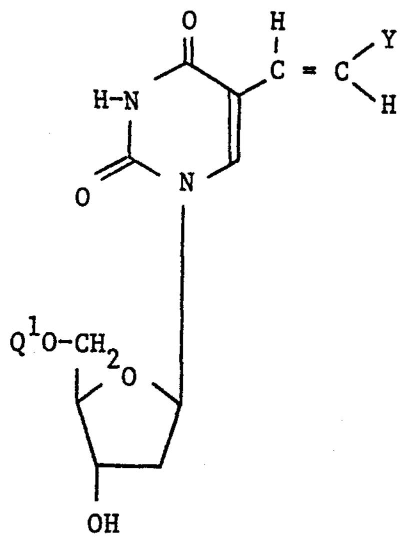

(IV)

wherein Y is as defined in formula (I) and $Q^1$ is an O-protecting group, with phosgene, suitably in an anhydrous polar organic solvent such as pyridine, and subsequently deprotecting the resulting product.

Compounds of formula (I) wherein $R^1$ and $R^2$ are hydrogen atoms and $R^3$ is a deoxyuridinylcarbonyl group, may be prepared by treating a compound of formula (V):

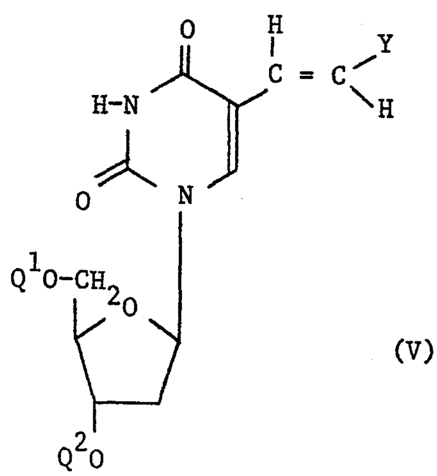

(V)

wherein Y is as defined in formula (I) and $Q^1$ and $Q^2$

are O-protecting groups, with phosgene, suitably in an anhydrous polar organic solvent such as pyridine, and subsequently deprotecting the resulting product.

Preferred O-protecting groups are trityl groups, such as 4,4'-dimethoxytrityl, or trialkylsilyl groups, such as trimethylsilyl. Deprotection may be carried out by treatment with an acid, such as aqueous acetic acid.

Compounds of formulae (IV) and (V) wherein $Q^1$ or $Q^2$ are trialkylsilyl groups may be prepared by treating a compound of formula (III) as defined above with a silylating agent. Suitable silylating agents are trialkylhalosilanes or N,O-bistrialkylsilylacetamides.

Compounds of formula (I) which are produced by the above reactions with phosgene, may be acylated by treating the products with a suitable acylating agent, preferably an agent of formula (VI):

$$X - \underset{\underset{O}{\|}}{C} - L \qquad (VI)$$

wherein X is as defined in formula (II), and L is a good leaving group. A preferred acylating agent is an acid anhydride.

The acylation reaction will generally produce a mixture of reaction products wherein one or more of $R^1$, $R^2$ and $R^3$ may be an acyl group, and the deoxyuridinylcarbonyl group may itself be substituted by one or more acyl groups. The extent of acylation will depend on a number of factors, such as the relative amounts and chemical natures of reactants, the physical conditions of the reaction, and the solvent system. The mixture

can be separated into its pure components by chromatographic methods.

A preferred acylation reaction comprises treating a compound of formula (I) wherein $R^1$ is a 5-(*E*-2-halogenovinyl)-2'-deoxyuridinyl carbonyl group, and $R^2$ and $R^3$ are each hydrogen, with an anhydride of a $C_{1-6}$ alkanoic acid. This reaction can lead to acylation at the 3'-position on the compound of formula (I) as well as at the 3'-position on the deoxyuridinyl carbonyl group. In addition, acylation at the 3-position on the deoxyuridinyl carbonyl group may occur under appropriate conditions.

Compounds of formula (I) in which $R^1$ is a deoxyuridinyl carbonyl group linked via an alkylene carbonyl group, and $R^2$ and $R^3$ are both hydrogen atoms, may be prepared by treating a compound of formula (III) as defined above with an activated dicarboxylic acid derivative of formula (VII):

$$L - \overset{\overset{\displaystyle O}{\|}}{C} - alk - \overset{\overset{\displaystyle O}{\|}}{C} - L \qquad (VII)$$

wherein 'alk' represents an alkylene moiety having from 1 to 8 carbon atoms, and L is a leaving group, suitably halogen or $-O-\overset{\overset{\displaystyle O}{\|}}{C}-OR^4$ where $R^4$ is $C_{1-6}$ alkyl. The reaction is suitably carried out in an anhydrous polar solvent, such as pyridine, and the product purified chromatographically.

The compounds of formula (I) may be formulated for use in a pharmaceutical composition. Accordingly, in a further aspect of the invention, there is provided a pharmaceutical composition which comprises a compound

of the formula (I) together with a pharmaceutically acceptable carrier or excipient.

Compositions which may be administered by the oral route to humans may be compounded in the form of syrups, tablets and capsules. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added.

The compounds may also be presented with a sterile liquid carrier for injection. The liquid carrier suitably may be a sterile oil or sterile water.

The composition may also be formulated for topical application to the skin or eyes.

For topical application to the skin, the compounds of the invention may be made up into a cream, lotion or ointment. These formulations may be conventional formulations well known in the art, for example, as described in standard books of pharmaceutics and cosmetics, such as Harry's Cosmeticology published by Leonard Hill Books and the British Pharmacopaeia.

The composition for application to the eyes may be a conventional eye-drop composition well known in the art.

Preferably, the compositions of this invention are in unit dosage form or in some other form that the patient may administer to himself a single dose. A suitable dosage unit might contain from 50 mg to 1 g of active ingredient, for example 100 to 500 mg. Such doses may be administered 1 to 4 times a day or more usually 2 or 3 times a day. The effective dose of compound depends on the particular compound employed, but is in general in the range of from 1.0 to 20 mg/kg of body weight per day or more usually 2.0 to 10 mg/kg per day.

The present invention further provides a method for treating non-human animals or humans suffering from viral infections, which comprises administering to the sufferer a pharmaceutically effective, non-toxic amount of a compound of formula (I):

The following Examples illustrate the invention.

Example 1

bis-[5-(E-2-Bromovinyl)-2'-deoxyuridin-5'-yl] carbonate

To a stirred solution of 5-(E-2-bromovinyl)-2'-deoxyuridine (5 g) in pyridine (100 ml) at -30°C, a solution of phosgene in toluene (12.5%, 7 ml) was added over 0.5h and then the mixture was allowed to come to room temperature and stirred at room temperature for 2h. The mixture was then partitioned between aqueous hydrochloric acid and ethyl acetate. The organic extract was washed with brine, aqueous sodium bicarbonate, dried, and the solvent removed. The residue was filtered through a column of silica gel (200 g). Elution with ether-acetone (2:1) gave the title compound (1.4 g), mp 216-222°C:

$\lambda_{max.}$ (EtOH) 250 ($\epsilon$ 28,400) and 293 ($\epsilon$ 23,300) nm;

$\nu_{max.}$(KBr) 1695 (broad), 1470 and 1382 $cm^{-1}$;

$\delta^1H$ (DMSO-$d_6$) 2.20 (4H, t, J=6Hz), 3.84-4.40 (8H, m), 4.45 (2H, m, exchanged with $D_2O$), 6.16 (2H, t, J=6Hz), 6.88 (2H, d, J=14Hz), 7.32 (2H, d, J=14Hz), 7.80 (2H, s) and 11.64 (2H, m, exchanged by $D_2O$);

(Found C, 39.9; H, 3.5; N, 7.85 %; $C_{23}H_{24}N_4O_{11}Br_2$ requires C, 39.9; H, 3.5; N, 8.1%).

Example 2

3',3'-O-3-Triacetyl-bis-[5-(E-2-bromovinyl)-2'-deoxyuridin-5'-yl] carbonate

and

Example 3

bis-[3'-O-Acetyl-5-(E-2-bromovinyl)-2'-deoxyuridin-5'-yl] carbonate

A solution of *bis*-[5-(*E*-2-bromovinyl)-2'-deoxyuridin-5'-yl] carbonate (0.8 g) in pyridine (10 ml) and acetic anhydride (2 ml) was stirred at room temperature overnight. The mixture was then partitioned between aqueous hydrochloric acid and ethyl acetate. The organic extract was washed with brine, aqueous sodium bicarbonate, dried, and the solvent removed. The residue was chromatographed over silica gel (40 g). Elution with ethyl acetate-hexane (4:1) gave 3',3'-O-3-triacetylbis-[5-(*E*-2-bromovinyl)-2'-deoxyuridin-5'-yl] carbonate (Example 2) (0.1 g);

$\lambda_{max.}$ (EtOH) 249 ($\varepsilon$ 14,100) and 293 ($\varepsilon$ 10,700) nm;

$\nu_{max.}$ ($CHCl_3$) 1800, 1750, 1715 and 1675 $cm^{-1}$;

$\delta$ $^1H$ ($CDCl_3$) 2.13 (6H, s), 2.00-2.60 (4H, m), 2.57 (3H, s), 4.35 (2H, m), 4.48 (4H, m), 5.26 (2H, m), 6.31 (2H, broad t, J=6Hz), 6.68 (2H, d, J=14Hz), 7.38 (1H, d, J=14Hz), 7.42 (1H, d, J=14Hz), 7.56 (1H, s), 7.60 (1H, s) and 9.58 (1H, broad s);

followed by *bis*-[3'-*O*-acetyl-5-(*E*-2-bromovinyl)-2'-deoxyuridin-5'-yl] carbonate (Example 3) (0.43 g),

$\lambda_{max.}$ 247 ($\varepsilon$ 26,500) and 285 ($\varepsilon$ 22,100) nm;

$\nu_{max.}$ (KBr) 1715, 1685 and 1237 $cm^{-1}$;

$\delta^1H$ ($CDCl_3$) 2.13 (6H, s), 2.00-2.70 (4H, m), 4.20-4.60 (6H, m), 5.25 (2H, m), 6.32 (2H, broad t, J=6Hz), 6.67 (2H, d, J=14Hz), 7.43 (2H, d, J=14Hz), 7.56 (2H, s) and 9.74 (2H, s, exchanged by $D_2O$).

(Found: C, 42.3; H, 3.85; N, 6.9%. $C_{27}H_{28}N_4O_{13}Br_2$ requires C, 41.75; H, 3.65; N, 7.2%).

0095292

Example 4

bis-[5-(E-2-Bromovinyl)-2'-deoxyuridin-3'-yl] carbonate

A solution of phosgene in toluene (12.5%, 3 ml) was added slowly over 0.5h to a solution of 5-(E-2-bromovinyl)-2'-deoxy-5'-O-(4,4'-dimethoxytrityl)-uridine (3 g) in pyridine (50 ml) at -30°C. The mixture was allowed to come to room temperature and then stirred at room temperature for 3h. The mixture was then partitioned between aqueous hydrochloric acid and ethyl acetate. The organic extract was washed with brine, aqueous sodium bicarbonate, dried, and the solvent removed. The residue was chromatographed over silica gel (150 g). Elution with dichloromethane-ethanol (97:3) gave bis-[5-(E-2-bromovinyl)-2'-deoxy-5'-O-(4,4'-dimethoxytrityl)uridin-3-yl] carbonate (a) (1.2 g):

$\nu_{max.}$ ($CHCl_3$) 1750, 1710, 1690 and 1250 $cm^{-1}$;

A solution of the compound (a) (1.2 g) in 80% aqueous acetic acid (30 ml) was stirred at room temperature for 2h. The mixture was then evaporated to dryness with the help of toluene. The residue was chromatographed over silica gel (50 g). Elution of the column with ethyl acetate-hexane (9:1) gave bis-[5-(E-2-bromovinyl)-2'-deoxyuridin-3'-yl] carbonate (0.3 g).

$\lambda_{max.}$ (EtOH) 249 ($\varepsilon$ 28,400) and 292 ($\varepsilon$ 24,800) nm;

$\delta^1H$ ($DMSO-d_6$) 2.36 (4H, m), 3.62 (4H, m), 4.10 (2H, m), 5.15 (2H, m, exchanged with $D_2O$), 5.20 (2H, m), 6.32 (2H, broad t, J=6Hz), 6.80 (2H, d, J=14Hz), 7.24 (2H, d, J=14Hz), 8.06 (2H, s) and 11.66 (2H, s, exchanged by $D_2O$).

Example 5

3,3-Carbonyl-*bis*-[5-(*E*-2-bromovinyl)-2'-deoxyuridine]

A solution of phosgene in toluene (12.5%, 1.02 ml) was added to a stirred solution of 5-(*E*-2-bromovinyl)-2'-deoxy-3',5'-*bis*-*O*-trimethylsilyluridine (1.02 g) and triethylamine (0.6 ml) in tetrahydrofuran (10 ml) at -30°C. The mixture was allowed to come to room temperature and stirred at room temperature for 2h. Further triethylamine (0.6 ml) and phosgene solution in toluene (12.5%, 1.02 ml) were added and the mixture was stirred at room temperature overnight. It was then partitioned between aqueous hydrochloric acid (2*M*)-ethyl acetate (1:1, 100 ml). The organic extract was washed with aqueous sodium bicarbonate, brine, dried and evaporated. The residue was chromatographed over silica gel (30 g). Elution of the column with ether-acetone (2:1) gave the title compound as microcrystals (0.08 g) which decomposed at 160°C without melting:

$\lambda_{max.}$ (EtOH) 250 ($\varepsilon$ 30,500) and 313 ($\varepsilon$ 14,200) nm;

$\nu_{max.}$ (KBr) 3520, 3430, 1809, 1732, 1695, 1500, 1388, 1058 and 530 $cm^{-1}$;

$\delta^{1}H$ [$(CD_3)_2SO$] 2.19 (4H, m), 3.66 (4H, m), 3.82 (2H, m), 4.26 (2H, m), 5.09-5.28 (4H, m, $D_2O$ exchangeable), 6.07 (2H, t, *J* 6Hz), 6.83 (2H, d, *J* 14Hz), 7.18 (2H, d, *J* 14Hz), and 8.24 (2H, s);

(Found: C, 40.2; H, 3.6; N, 7.7%. $C_{23}H_{24}Br_2N_4O_{11}$ requires: C, 39.9; H, 3.5; N, 8.1%).

0095292

Example 6

bis-[5-(E-2-Bromovinyl)-2'-deoxyuridin-5'-yl] succinate

Succinyl chloride (0.7ml) in dichloromethane (5ml) was added slowly to a stirred solution of 5-(E-2-bromovinyl)-2'-deoxyuridine (1.8g) in pyridine (20ml) at 0°C and the mixture was then stirred at room temperature overnight. It was then partitioned between aqueous hydrochloric acid and ethyl acetate. The organic extract was washed with brine, aqueous sodium bicarbonate, dried, and evaporated. The residue was chromatographed over silica gel (60g). Elution of the column with ether-acetone (3:2) gave the title compound as an amorphous powder (0.15g),

$\lambda_{max.}$ (EtOH) 249 ($\varepsilon$ 26,400) and 293 ($\varepsilon$ 22,300) nm;

$\nu_{max.}$ (KBr) 1710 (broad), 1468 and 1282 $cm^{-1}$;

$\delta^1H$ [$(CD_3)_2SO$] 2.17 (4H, t, J 6Hz, 2'-$CH_2$), 2.60 (4H, s, $-O_2CCH_2CH_2CO_2-$), 3.90 (2H, m, 4'-CH), 4.22 (6H, m, 5'-$CH_2$, 3'-CH), 5.37 (2H, m, $D_2O$ exchangeable, OH), 6.13 (2H, t, J 6Hz, 1'-CH), 6.88 (2H, d, J 14Hz, CH=CHBr), 7.28 (2H, d, J 14Hz, CH=CHBr), 7.74 (2H, s, 6-CH), and 11.55 (2H, m, $D_2O$ exchangeable, NH);

Example 7

bis-[5-(E-2-Bromovinyl)-2'-deoxyuridin-5'-yl] sebacate

Sebacyl chloride (1.3ml) in dichloromethane (5ml) was added slowly to a stirred solution of 5-(E-2-bromovinyl)-2'-deoxyuridine (1.94g) in pyridine (20ml) at 0°C and the mixture was then stirred at room temperature overnight. It was then partitioned between ethyl acetate and water. The organic extract was washed with aqueous hydrochloric acid, brine, aqueous sodium bicarbonate, dried, and evaporated. The residue was chromatographed over silica gel (80g). Elution of the column with dichloromethane-ethanol (9:1) gave the title compound (0.3g), mp 164-170°C (from ethanol);

$\lambda_{max.}$ (EtOH) 250 ($\varepsilon$ 24,700) and 293 ($\varepsilon$ 22,100) nm;

$\nu_{max.}$ (KBr) 1700 (broad), 1468, 1280 and 1090 $cm^{-1}$;

$\delta^1H$ [$(CD_3)_2SO$] 1.05-1.70 (12H, m), 2.08-2,45 (8H, m), 3.92 (1H, m, 4'-CH), 4.21 (6H, m, 5'-$CH_2$, 3'-CH), 5.40 (2H, m, $D_2O$ exchangeable, OH), 6.15 (2H, t, J 6Hz, 1'-CH), 6.90 (2H, d, J 14Hz, CH=CHBr), 7.76 (2H, s, 6-CH), and 11.55 (2H, m, $D_2O$ exchangeable, NH);

(Found: C, 45.15; H, 4.75; N, 6.45%. $C_{32}H_{40}N_4O_{12}Br_2$ requires C, 45.2; H, 4.95; N, 6.6%.)

Antiviral Activity

In Vitro

Method

Vero (African Green Monkey Kidney) cells were grown to confluence in 24 well multidishes, each well being 1.6 cm in diameter. The cells were infected with Herpes simplex type 1 virus (HFEM strain) and overlaid with 0.5 mL of 0.9% agarose (w/v) in maintenance medium. Test compounds prepared in maintenance medium in concentrations ranging from 200 to 0.06 µg/mL in half-log dilution steps, were added in 0.5 mL volume. The virus infected cultures were then incubated at 37°CC for 4 days before fixing in 4% formaldehyde solution and staining with carbolfuchsin. The dishes were then examined to find what concentration of test compound caused a 50% reduction in the number of virus plaques formed ($PDD_{50}$ value) and the minimum concentration of test compound which caused cytotoxicity (MTD).

Results

| Example No | $PDD_{50}$ | | MTD (µg/mL) |
|---|---|---|---|
| | µg/ml | µM | |
| 1 | 1.5 | 2.1 | 100 |
| 2 | 12.4 | 15.1 | 100 |
| 3 | 8.5 | 10.9 | 100 |
| 4 | 0.34 | 0.5 | 100 |
| 5 | 0.07 | 0.1 | 100 |
| 6 | 4.8 | 6.4 | 100 |
| 7 | 0.12 | 0.14 | 100 |

CLAIMS

1. A compound of formula (I):

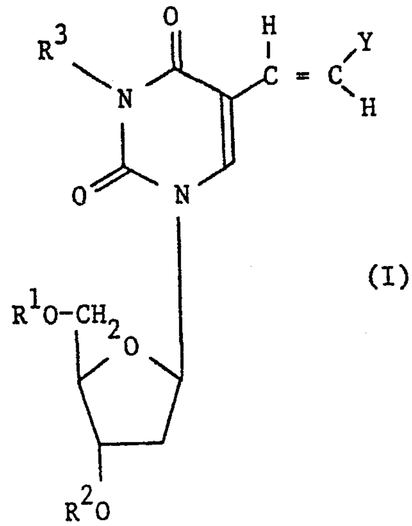

(I)

wherein each of $R^1$, $R^2$ and $R^3$ is a hydrogen atom, an acyl group, or an optionally acylated 5-(E-2-halogenovinyl)-2'-deoxyuridinylcarbonyl group, or $R^1$ is a 5-(E-2-halogenovinyl)-2'-deoxyuridinylcarbonyl group linked to the 5'-oxygen atom via an alkylene-carbonyl group having from 1 to 8 carbon atoms in the alkylene moiety, and Y is a halogen atom, identical to the halogen atom in the deoxyuridinylcarbonyl or linked deoxyuridinylcarbonyl group, with the provisos that, (a) only one of $R^1$, $R^2$ and $R^3$ is an optionally acylated 5-(E-2-halogenovinyl)-2'-deoxyuridinylcarbonyl group, or (b) $R^1$ is a 5-(E-2-halogenovinyl)-2'-deoxyuridin-5'-ylcarbonyl group linked via the alkylene-carbonyl group and $R^2$ and $R^3$ are both simultaneously hydrogen atoms.

2. A compound according to claim 1, in which Y represents a bromine atom.

3. A compound according to claim 1 or 2, in which $R^1$, $R^2$, or $R^3$ is an acyl group of formula (II):

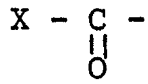

(II)

wherein X is an alkyl, aryl, or aralkyl group.

4. A compound according to claim 3, in which X is a $C_{1-6}$ alkyl, optionally substituted phenyl, or optionally substituted benzyl group.

5. A compound according to any one of claims 1 to 4, in which the alkylene-carbonyl group is a straight chain alkylene group.

6. A compound according to any one of claims 1 to 5, in which $R^2$ and $R^3$ are both hydrogen atoms.

7. A process for preparing a compound according to claim 1, which comprises reacting an optionally <u>O</u>- protected compound of formula (III):

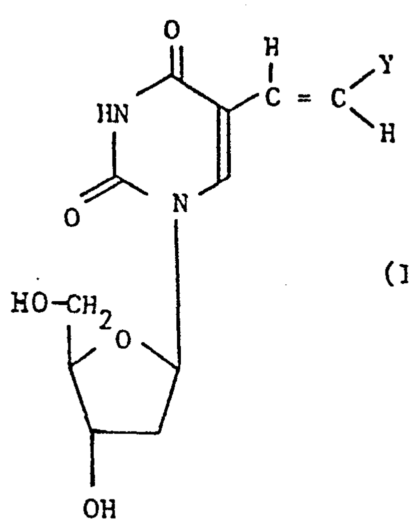

(III)

wherein Y is as defined in claim 1,

with phosgene or a compound of formula (VII):

$$L - \overset{O}{\overset{\|}{C}} - alk - \overset{O}{\overset{\|}{C}} - L \qquad (VII)$$

wherein 'alk' represents an alkylene moiety having from 1 to 8 carbon atoms, and L is a leaving group.

8. A process for preparing a compound according to claim 1, which comprises reacting a compound of formula (I) as claimed in claim 1 with an acylating agent of formula (VI):

$$X - \underset{O}{\underset{\|}{C}} - L \qquad (VI)$$

wherein X is as defined in claim 1 and L is a leaving group.

9. A pharmaceutical composition comprising a compound according to any one of claims 1 to 6 in combination with a pharmaceutically acceptable carrier.

10. A compound according to any one of claims 1 to 6, or a composition according to claim 9, for use in the treatment of viral infections.

European Patent Office

# EUROPEAN SEARCH REPORT

0095292
Application number

EP 83 30 2735

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,Y | DE-A-2 915 254 (UNIVERSITY OF BIRMINGHAM)<br>* Pages 1,2 *<br>--- | 1,6,9, 10 | C 07 H 19/06<br>C 07 H 19/08<br>A 61 K 31/70 |
| X,Y | DE-A-3 010 399 (K. GAURI)<br>* Pages 5-7 *<br>--- | 1-4,6, 9,10 | |
| Y,P | DE-A-3 129 341 (ROBUGEN GmbH)<br>* Pages 1,2 *<br>--- | 1,6,9, 10 | |
| X,Y D | EP-A-0 061 283 (BEECHAM)<br>* Pages 1-4, claims *<br>----- | 1-4,6, 9,10 | |
| | | | TECHNICAL FIELDS SEARCHED (Int. Cl. 3) |
| | | | C 07 H 19/00<br>A 61 K 31/00 |

The present search report has been drawn up for all claims

| Place of search | Date of completion of the search | Examiner |
|---|---|---|
| THE HAGUE | 26-08-1983 | VERHULST W. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82